# EUROPEAN PATENT SPECIFICATION

(11) **EP 1 440 990 B1**
(45) Date of publication and mention of the grant of the patent: **15.08.2007**
(21) Application number: 04462002.9
(22) Date of filing: 26.01.2004
(51) Int. Cl.: C08G 18/08, C08G 18/76, C07C 263/10, C07C 263/20

(54) **Process for decreasing the acidic content of polymethylene-poly-phenyl-polyisocyanates mixtures**
Verfahren zur Reduzierung des Säuregehaltes von Polymethylen-Polyphenyl-Polyisozyanat Mischungen
Procédé pour diminuer l'acidité de mélanges de polyéthyléne-polyphényl-polyisocyanates

(30) Priority: 27.01.2003 HU 0300226
(43) Date of publication of application: 28.07.2004
(73) Proprietor: BorsodChem Rt., 3702 Kazincbarcika (HU)
(72) Inventor: Kovacs, Laszlo F., 3700 Kazincbarcika (HU); Purzsa, Tamas, 3700 Kazincbarcika (HU); Kozar, Zoltan Dr., 3700 Kazincbarcika (HU); Toth, Andras Dr., 3529 Miskolc (HU); Fekete, Laszlo, 3700 Kazincbarcika (HU); Diczhazi Szilagyi, Szilvia, 3630 Putnok (HU); Trujillo Vilaboy, José, 3525 Miskolc (HU); Janusz Marcin, Szojka, 3300 Eger (HU)
(74) Representative: Kerény, Judit

(56) References cited:
- EP-A- 0 464 704
- GB-A- 1 458 747
- US-A- 3 857 871
- US-A- 3 925 437
- US-A- 4 118 286
- US-A- 6 127 463

## Description

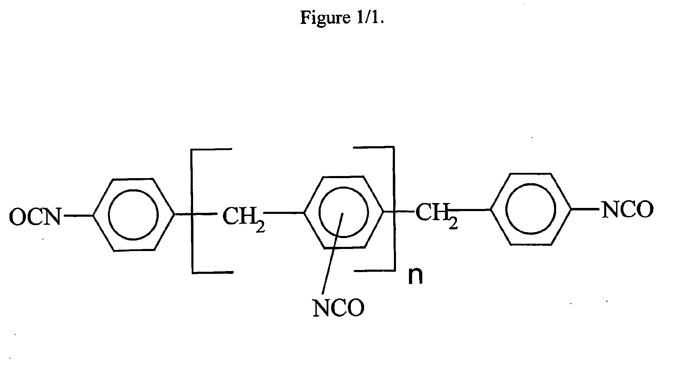

This invention aims at the decrease of acidic content of polymethylene-poly-phenyl-polyisocyanates mixtures (PMDI). The chemical formula of PMDI (n=0-5), is shown in Figure 1. Polymethylene-poly-phenyl-polyamine (PMDA) is made by the reaction of aniline and formaldehyde in the presence of an acidic catalyst, and subsequently PMDI is produced by the phosgenation of PMDA mixture in the presence of a neutral solvent. PMDI is widely used for the production of polyurethane foams, binders, adhesives and similar products.

A minor portion of HCl which is formed as a by-product in the phosgenation reaction of PMDI production process remains as impurity in the product even after the removal of phosgene and solvent - which were present in excessive quantities in the production process. Normally, the acidic content of the product PMDI is 150-250 wppm. The acidic impurity of the product PMDI blocks the alkaline catalysts used for promoting the formation of polyurethanes, thus it negatively affects the speed of polyurethane formation, in many cases it also has an impact on the properties of the processed foam.

Due to the above acidic content of the PMDI used for polyurethane production should not be too high, therefore after the removal of phosgene and solvent from PMDI, the acidic content has also to be decreased in steps below 100 wppm. This is normally achieved by passing nitrogen through the mixture while its temperature is increased and the pressure is decreased. The disadvantage of the process is that due to the elevated temperature side reactions take place in the PMDI, which also lead to colorization.

Similar physical processes for decreasing the acidic content are disclosed in US 3.857.871 and US 4.118.286. Chemical processes are not so often used in the industrial practice such as disclosed in GB 1.458 747, US 6.127-463 and US 3.925.437., wherein organic compounds are applied in the coarse of the process.

The subject of the patent is a process for the decrease of the acidic content of PMDI. It employs relatively low temperature, its time requirement is similar to the process in use, and it removes the sufficient amount of acidic impurities from the mixture by the application of a mineral-based acid sorption material. The main advantage of the process that no colorization or minimum colorization takes place at the temperature employed, so the required decrease of the acidic content will be realized without the deterioration of PMDI color, to the contrary of the process employing heat treatment.

In the patented process the solid acid sorption material of mineral origin or synthetic one having large specific surface area is contacted with PMDI at the appropriate temperature through a defined period of time, in equipment specifically prepared for that purpose.

This acid sorption material is an alkaline mineral with large specific surface area or a synthetically produced inorganic compound, which can be used in the form of powder, granule or other forms of similar nature. This material - under the process conditions - will not solve in PMDI and will not react with it. For the above purpose suitable materials are: aluminium and/or magnesium compounds containing carbonate and/or hydroxide groups, meeting the criteria. Such a material is Mg×Al₂(OH)_{y}CO₃ * z H₂O (x=1-6, y=6-16, z=3-4), called Hidrotalcit, which is widely used for acid sorption applications in the field of plastics.

In the patented process the acid sorption material should be contacted with PMDI. That could be implemented by suspending the acid sorption material in PMDI, then intensely stirring. Alternatively, PMDI can be passed through an appropriate column or filter layer at adequate temperature and contact time, where the acid sorption material is present as packing, filtering agent, independently or mixed with inert materials (e.g. silicates).

The acid sorption material can be advantageously used in powder or granule form. In such case, the treatment can be implemented by adding 0.1-1 wt. %, preferably 0.03-0.3 wt.% acid sorption material into PMDI in the temperature range of 20-105°C, preferably 40-80°C, and subsequent stirring of the mixture at a constant temperature. The stirring time is 10-300 minutes, preferably 40-240 minutes. When the stirring is completed, the acid sorption material is removed by simply using one of the standard filtering processes.

The process invented can be realized in any equipment good for effective contacting solid or liquid phases and their subsequent separation; that is in any agitated equipment with appropriate filter, any packed column, or in a given case any simple filter unit.

In the following illustrative examples, the determination of acidic content was made by titrimetry method, while the color of PMDI was measured by photometry:

### Example no. 1. :

0.2 g Hidrotalcit powder is made up in a 300 ml Erlenmeyer-flask, then we add 200 g PMDI of 220 ppm acidic content, L=29,00, a=38,86, b=48,96 color indexes. Then the mixture is stirred on a hot plate equipped with stirrer at 50°C, 500 rpm speed maintained through 3 hours, and finally it is filtered on a vacuum filter. The acidic content of the material after removal of the acid sorption material is 96 ppm, while the color indexes are: L=33,61, a=42,13, b=57,00.

### Example no. 2. :

Vacuum filter equipment with 10 cm dia. glass filter surface is used for preparing the filter bed by mixing 1 g Hidrotalcit acid sorption material and 10 g inert mineral. 650 g PMDI of 155 ppm acidic content and color indexes of L=19,19, a=36,74, b=31,00 is filtered through in the presence of N₂ as protection gas. The same operation is repeated twice on the same filter bed. The gained PMDI has the following characteristics: 93 ppm acidic content, color indexes are: L=19,66, a=36,91, b=31,75.

### Comperative example no. 1. :

In a four-neck flask of 500 ml, which is equipped with thermometer, mechanical stirrer and N₂ supply, 200 g PMDI sample of 220 ppm acidic content and color indexes L=29,00, a=38,86, b=48,96 (the same sample as used in example no. 1) is filled in. A vacuum pump is connected to the flask. 200 ml/min nitrogen is passed through the mixture through 2 hours while 200°C temperature is maintained. At the end of the treatment the acidic content of PMDI is 94 ppm, color indexes are: L=13,87, a=30,76, b=23,36.

## Claims

1. Process for the production of polymethylene-poly-phenyl-isocyanate (PMDI) mixtures with maximum acidic content of 100 ppm, wherein PMDI is resulted from the reaction of phosgene and polymethylene-poly-phenylpolyamine mixtures, subsequently excess phosgene and solvent is removed from PMDI and this material is treated with a chemical acid-binding solid alkaline Mg-Al-hydroxy-carbonate of mineral origin or its synthetically produced form.

2. Process of claim 1, wherein the treatment takes place at a temperature of 20-105 °C by contacting at a great surface PMDI and chemical acid- binding solid alkaline Mg-Al-hydroxy-carbonate of mineral origin or its synthetically produced form and their subsequent separation.

3. Process of claim 1 wherein given quantity of PMDI is treated with 0.01-1 % by weight of chemical acid- binding solid alkaline Mg-Al-hydroxy-carbonate of mineral origin or its synthetically produced form.

## Patentansprüche

1. Verfahren zur Herstellung von Polymethylen-polyphenyl-isocyanat (PMDI) Gemischen mit einem maximalen Säureinhalt von 100 ppm, **dadurch gekennzeichnet, dass** man das PMDI durch die Umsetzung von Phosgen mit Polymethylen-poly-phenyl-polyamin Gemischen erhält, wonach man den Überschuss von Phosgen und das Lösungsmittel aus dem PMDI entfernt und dieses Material mit einem chemischen säurebindenden festen alkalischen Mg-Al-hydroxycarbonat mineralischer Herkunft oder mit seiner synthetisch hergestellten Form behandelt.

2. Verfahren nach Anspruch 1, **dadurch gekennzeichnet, dass** man die Behandlung bei 20-105 °C durch die Kontaktierung von PMDI an einer grossen Oberfläche mit einem festen säurebindenden alkalischen Mg-Al-hydroxy-carbonat von mineralischer Herkunft oder mit dessen synthetisch hergestellter Form und durch dessen nachfolgende Trennung durchführt.

3. Verfahren nach Anspruch 1 **dadurch gekennzeichnet dass** man eine gegebene Menge von PMDI mit 0.01 Gew.%-1 Gew. % von einem festen säurebindenden alkalischen Mg-Al-hydroxy-carbonat von mineralischer Herkunft oder mit dessen synthetisch hergestellter Form behandelt.

## Revendications

1. Procédé de préparation de mélanges polyméthylène-polyphenyl-isocyanate (PMDI) à teneur en acides inférieure ou égal à 100 ppm, **caractérisé en ce que** le PMDI résulte de la réaction du mélange phosgène et polyméthylène-polyphenyl-polyamine, après laquelle l'excès de phosgène et de solvant sont retirés du PMDI et ce dernier est traité par un capteur chimique d'acide qui est un Mg-Al-hydroxy-carbonate solide, alcalin, d'origine minérale ou produit synthétiquement.

2. Procédé selon la revendication 1, **caractérisé en ce que** le traitement a lieu à une température de 20 à 105 °C par mise en contact, avec une grande surface, le PMDI et le capteur chimique d'acide Mg-Al-hydroxy-carbonate solide, alcalin, d'origine minérale ou produit synthétiquement et puis par leur séparation.

3. Procédé selon la revendication 1, **caractérisé en ce qu'**une quantité donnée de PMDI est traitée par 0,01 à 1 % en poids du capteur chimique d'acide Mg-Al-hydroxy-carbonate solide, alcalin, d'origine minérale ou produit synthétiquement.
